# EUROPEAN PATENT APPLICATION

(11) **EP 3 763 830 A1**
(43) Date of publication of application: **13.01.2021**
(21) Application number: 19764446.1
(22) Date of filing: 05.03.2019
(51) Int. Cl.: C12Q 1/689, C12Q 1/6851, A23L 33/135, A61K 35/74, A61P 35/00, A61P 37/00

(54) **NANOVESICLES DERIVED FROM LACTOBACILLUS SP. BACTERIA, AND USE THEREOF**

(30) Priority: 05.03.2018 KR 20180026039; 04.03.2019 KR 20190024891
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2019/002500
(87) International publication number: WO 2019/172598

(57) **Abstract**

The present invention relates to vesicles derived from bacteria of the genus *Lactobacillus* and a use thereof, and the inventors experimentally confirmed that the vesicles were significantly reduced in samples obtained from patients with stomach cancer, renal failure, dementia and stroke, compared with a normal individual, and the vesicles inhibited the secretion of inflammatory mediators caused by pathogenic vesicles, was significantly inhibited. Therefore, it is expected that the vesicles derived from bacteria of the genus *Lactobacillus* according to the present invention can be effectively used for a method of diagnosing stomach cancer, renal failure, dementia or stroke, and for developing a composition for preventing, alleviating or treating an inflammatory disease accompanied by immune dysfunction, including the diseases.

<110>    MD Healthcare Inc.

<120>    Nanovesicles derived from Lactobacillus bacteria and Use thereof

<130>    MPCT19-033

<150>    KR 10-2018-0026039
<151>    2018-03-05

<150>    KR 10-2019-0024891
<151>    2019-03-04

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                    50


<210>    2
<211>    55
<212>    DNA
<213>    Artificial Sequence

<220>
<223>    16S_V4_R


<400>    2
gtctcgtggg ctcggagatg tgtataagag acaggactac hvgggtatct aatcc              55

## Description

### [Technical Field]

The present invention relates to nanovesicles derived from bacteria of the genus *Lactobacillus* and a use thereof, and more particularly, to a method of diagnosing stomach cancer, renal failure, dementia or stroke using nanovesicles derived from bacteria of the genus *Lactobacillus,* and a composition for preventing, alleviating or treating the disease, which comprises the vesicles.

Since the beginning of the 21st century, acute infectious diseases recognized as epidemic diseases in the past have become less important, whereas chronic inflammatory diseases accompanied by immune dysfunction caused by disharmony between humans and microbiomes have changed disease patterns as main diseases that determine the quality of life and the human lifespan. Particularly, metabolic diseases such as cancer and diabetes caused by westernization of dietary habits, cardiovascular diseases, such as myocardial infarction and stroke, neuropsychiatric diseases, such as Parkinson's disease, dementia and depression are becoming major problems for national health.

Such diseases are characterized by chronic inflammation, and chronic inflammation is accompanied by immune dysfunction against an external causative factor. As an immune response against a causative factor derived from bacteria, an immune response of Th17 secreting the interleukin (IL)-17 cytokine is important, and when exposed to a bacterial causative factor, neutrophilic inflammation caused by the Th17 immune response occurs. In the process of inflammation, an inflammatory mediator such as tumor necrosis factor-alpha (hereinafter referred to as TNF-α) plays an important role. In addition, it was recently reported that IL-6 secreted by a bacterial causative factor plays an important role in differentiation to Th17 cells, and is also involved in the pathogenesis of a cardiovascular disease or brain/neuropsychiatric disease.

Symbiotic microorganisms that live in the human body amount to 10² trillion, which are 10-fold more than human cells, and the number of genes in the microorganisms is known to be over 100 times that of human genes. Microbiota or microbiomes refer to a microbial community including bacteria, archaea and eukarya, present in a given habitat, and it is known that gut microbiota play an important role in human physiology, and have great effects on human health and diseases due to interaction with human cells.

Bacteria and archaea coexisting in our body and bacteria present in the ambient environment secrete nanometer-sized vesicles in order to exchange information on genes, proteins, and the like with other cells. The mucosa forms a physical defense membrane through which particles having a size of 200 nanometers (nm) or more cannot pass, so that bacteria coexisting in the mucosa cannot pass through the mucosa, but vesicles derived from bacteria have a size of 100 nanometers or less and are absorbed into our bodies after relatively freely passing through epithelial cells via the mucosa. It has recently been revealed that pathogenic bacteria-derived vesicles absorbed in the human body play an important role in the pathogenesis of metabolic diseases such as diabetes and obesity.

Bacteria of the genus *Lactobacillus* are gram-positive bacteria secreting lactic acid, and various bacteria belonging to the genus *Lactobacillus* are used as a probiotic product. However, there have been no reports on diagnostic and therapeutic techniques for diseases such as stomach cancer, renal failure, dementia and stroke using vesicles derived from bacteria of the genus *Lactobacillus* yet.

### [Disclosure]

### [Technical Problem]

As a result of earnest research to solve the conventional problems, the inventors confirmed that the content of vesicles derived from bacteria of the genus *Lactobacillus* significantly decreased in samples derived from patients with stomach cancer, renal failure, dementia and stroke, compared with a normal person, through metagenomic analysis, and also confirmed by isolating vesicles derived from bacteria of the genus *Lactobacillus in vitro* and evaluating therapeutic efficacy, an immunomodulatory effect was exhibited. Based on this, the present invention was completed.

Thus, an object of the present invention is to provide a method of providing information for diagnosis of with stomach cancer, renal failure, dementia or stroke.

In addition, the present invention is also directed to providing a composition for preventing, alleviating or treating an inflammatory disease accompanied by immune dysfunction, which comprises *Lactobacillus-derived* vesicles as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a method of providing information for diagnosing stomach cancer, renal failure, dementia or stroke, the method comprising the following steps:
(a) extracting DNAs from extracellular vesicles isolated from samples of a normal individual and a subject;
(b) performing polymerase chain reaction (PCR) on the extracted DNA using a pair of primers prepared based on a gene sequence present in 16S rDNA to obtain each PCR product; and
(c) classifying a case in which a content of extracellular vesicles derived from bacteria of the genus *Lactobacillus* is lower than that of the normal individual sample, as stomach cancer, renal failure, dementia or stroke, through quantitative analysis of the PCR product.

In addition, the present invention provides a method of diagnosing stomach cancer, renal failure, dementia or stroke, the method comprising the following steps:
(a) extracting DNAs from extracellular vesicles isolated from samples of a normal individual and a subject;
(b) performing polymerase chain reaction (PCR) on the extracted DNA using a pair of primers prepared based on a gene sequence present in 16S rDNA to obtain each PCR product; and
(c) determining a case in which a content of extracellular vesicles derived from bacteria of the genus *Lactobacillus* is lower than that of the normal individual sample, as stomach cancer, renal failure, dementia or stroke, through quantitative analysis of the PCR product.

As an embodiment of the present invention, the sample in Step (a) may be blood.

As another embodiment of the present invention, the primer pair in Step (b) may be primers of SEQ ID Nos. 1 and 2.

In addition, the present invention provides a pharmaceutical composition for preventing or treating an inflammatory disease accompanied by immune dysfunction, comprising vesicles derived from bacteria of the genus *Lactobacillus* as an active ingredient.

In addition, the present invention provides a food composition for preventing or alleviating an inflammatory disease accompanied by immune dysfunction, comprising vesicles derived from bacteria of the genus *Lactobacillus* as an active ingredient.

In addition, the present invention provides a cosmetic composition for preventing or alleviating an inflammatory skin disease, comprising vesicles derived from bacteria of the genus *Lactobacillus* as an active ingredient.

In addition, the present invention provides a use of vesicles derived from bacteria of the genus *Lactobacillus* for preventing or treating an inflammatory disease accompanied by immune dysfunction.

In addition, the present invention provides a method of preventing or treating an inflammatory disease accompanied by immune dysfunction, the method comprising a step of administering a pharmaceutical composition comprising vesicles derived from bacteria of the genus *Lactobacillus* as an active ingredient to a subject.

In one embodiment of the present invention, the vesicles may have an average diameter of 10 to 200 nm.

In another embodiment of the present invention, the vesicles may be secreted naturally or artificially from bacteria of the genus *Lactobacillus.*

In still another embodiment of the present invention, the vesicles derived from bacteria of the genus *Lactobacillus* may be one or more vesicles selected from the group consisting of *Lactobacillus* brevis-derived vesicles, *Lactobacillus* casei-derived vesicles, *Lactobacillus rhamnosus-derived* vesicles and *Lactobacillus* sakei-derived vesicles.

In yet another embodiment of the present invention, the inflammatory disease may be one or more diseases selected from the group consisting of stomach cancer, renal failure, dementia and stroke.

### [Advantageous Effects]

The inventors confirmed that intestinal bacteria are not absorbed into the body through epithelial cells, but bacteria-derived vesicles are absorbed, systemically distributed and then excreted out of the body through the kidneys, liver and lungs, and by metagenomic analysis for vesicles derived from bacteria present in patients' blood, also confirmed that vesicles derived from bacteria of the genus *Lactobacillus,* which are present in sample of patients with stomach cancer, renal failure, dementia and stroke significantly decrease, compared with a normal individual. In addition, when *Lactobacillus brevis, Lactobacillus casei, Lactobacillus rhamnosus,* and *Lactobacillus sakei* strains, which belong to bacteria of the genus *Lactobacillus,* are isolated and cultured in vitro, and then vesicles were isolated from the strains and administered to inflammatory cells, the secretion of inflammation mediators such as IL-6 and TNF-α by pathogenic vesicles was significantly inhibited, thereby confirming an immunomodulatory function, and the *Lactobacillus-derived* vesicles according to the present invention are expected to be effectively used for a method of diagnosing stomach cancer, renal failure, dementia or stroke, and a composition for preventing, alleviating or treating the disease.

### [Description of Drawings]

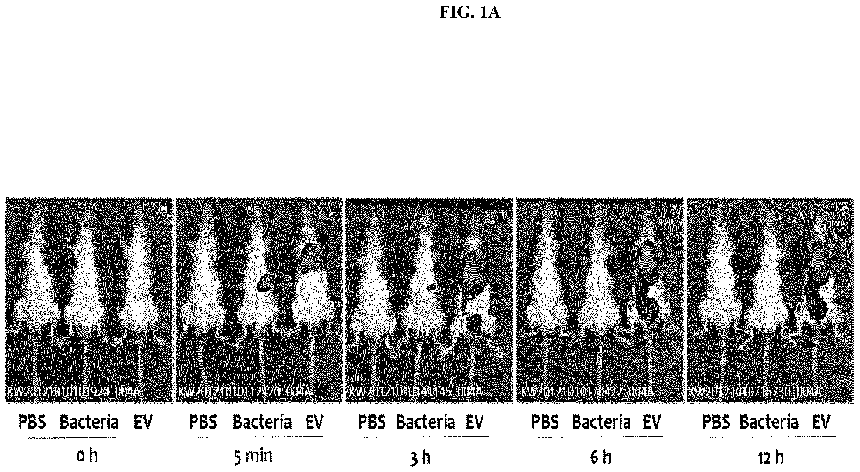

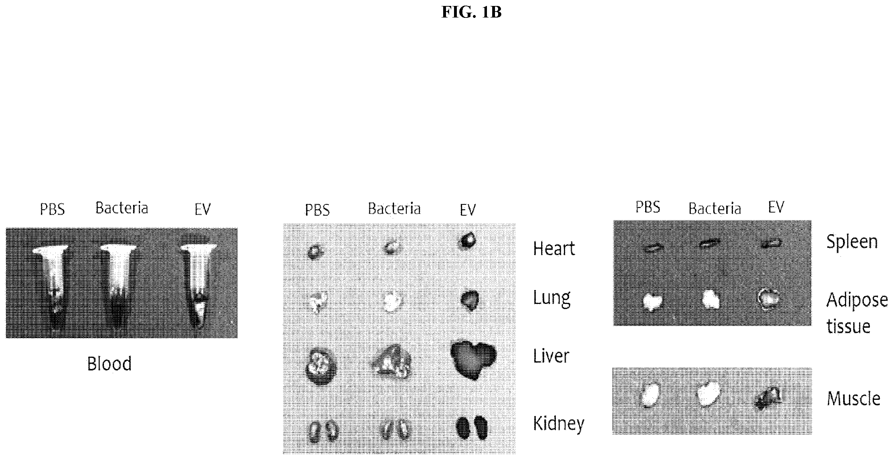

FIG. 1A is a series of photographs capturing distribution patterns of bacteria and bacteria-derived vesicles (EV) by time after the bacteria and the vesicles derived from bacteria were orally administered to mice, and FIG. 1B is a result of evaluating the in vivo distribution patterns of the bacteria and the vesicles by harvesting blood, kidneys, liver, and various organs at 12 hours after orally administering the bacteria and the vesicles.

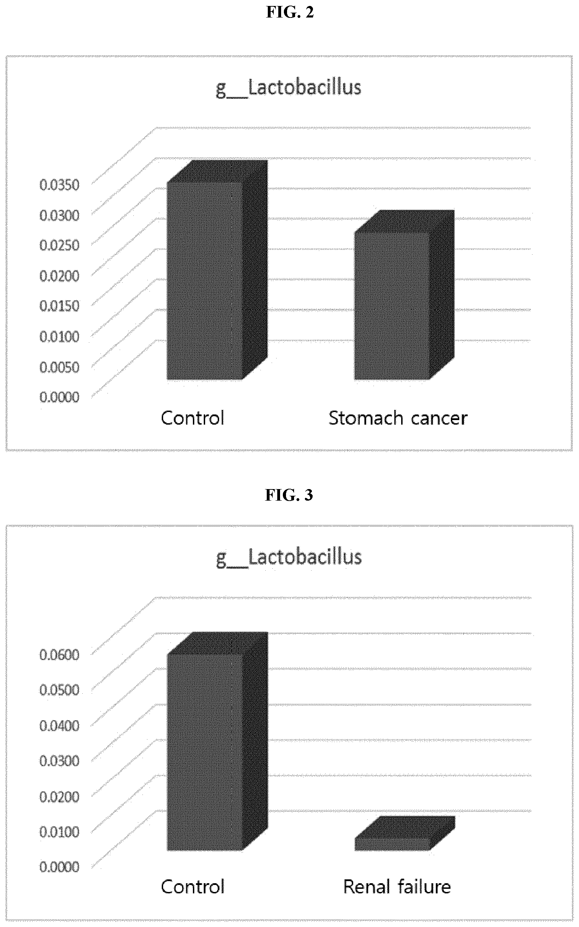

FIG. 2 is a result of comparing the distributions of vesicles derived from bacteria of the genus *Lactobacillus* after metagenomic analysis of bacteria-derived vesicles present in the blood of stomach cancer patients and a normal individual.
FIG. 3 is a result of comparing the distributions of vesicles derived from bacteria of the genus *Lactobacillus* after metagenomic analysis of bacteria-derived vesicles present in the blood of renal failure patients and a normal individual.

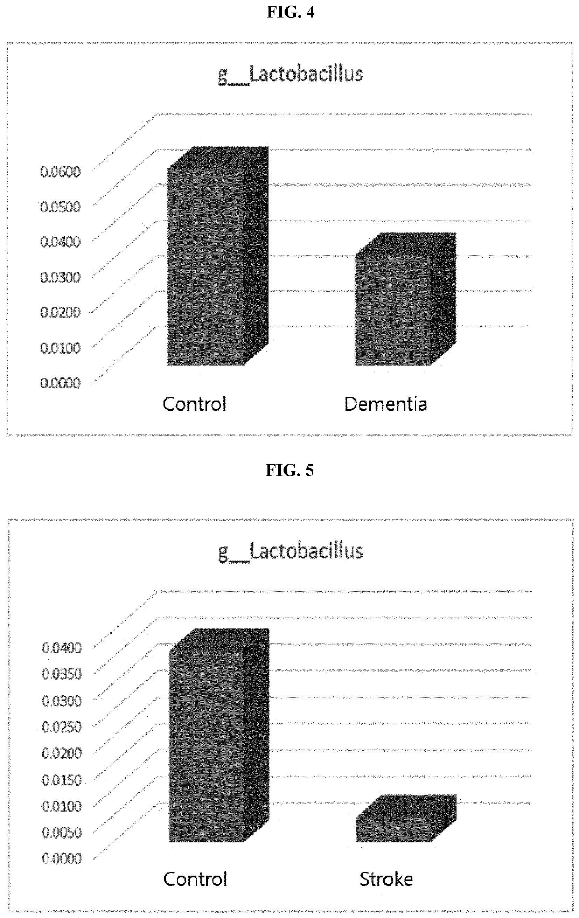

FIG. 4 is a result of comparing the distributions of vesicles derived from bacteria of the genus *Lactobacillus* after metagenomic analysis of bacteria-derived vesicles present in the blood of dementia patients and a normal individual.
FIG. 5 is a result of comparing the distributions of vesicles derived from bacteria of the genus *Lactobacillus* after metagenomic analysis of bacteria-derived vesicles present in the blood of stroke patients and a normal individual.

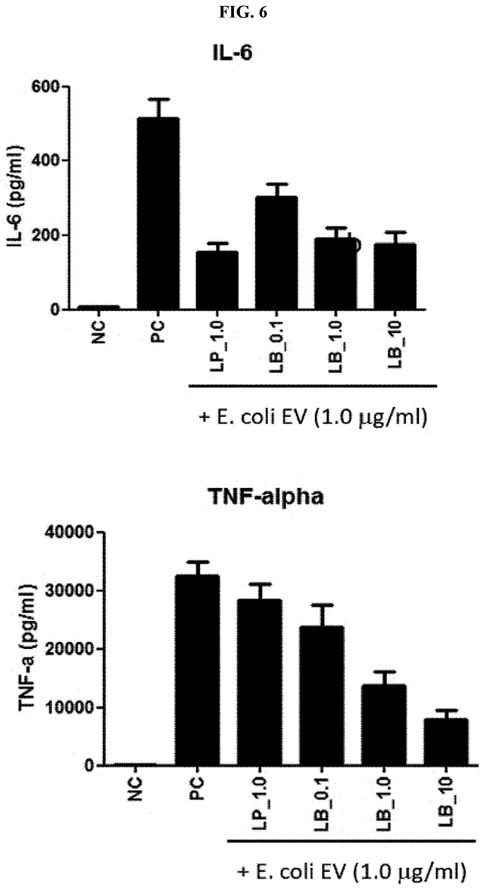

FIG. 6 is a result of evaluating an effect on the secretion of IL-6 and TNF-α which inflammatory mediators caused by *E. coli* EVs by pretreating vesicles derived from *Lactobacillus plantarum (L. plantarum)* or *Lactobacillus brevis* (*L. brevis*) before treatment of pathogenic vesicles such as *E. coli* EVs to evaluate immunomodulatory and anti-inflammatory effects of *Lactobacillus* brevis-derived vesicles (PC: positive control; LP: *Lactobacillus plantarum* EVs; LB: *Lactobacillus brevis* EVs).

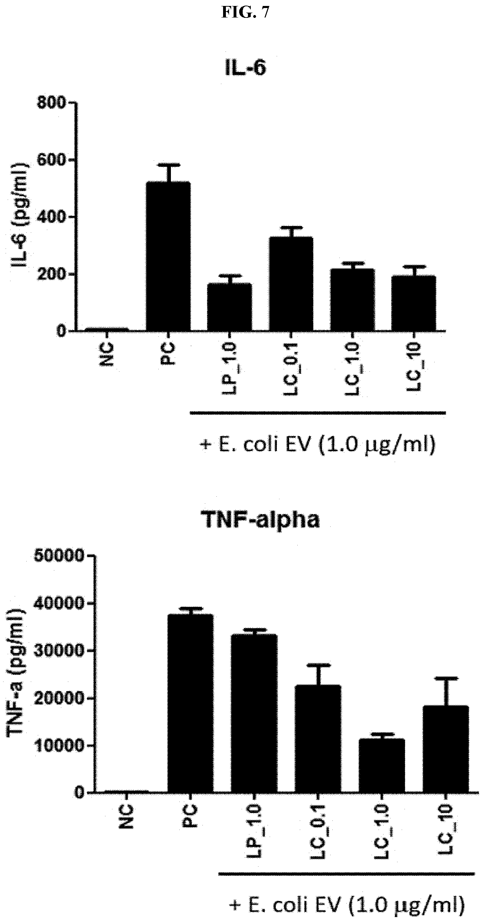

FIG. 7 is a result of evaluating an effect on the secretion of IL-6 and TNF-α which inflammatory mediators caused by *E. coli* EVs by pretreating vesicles derived from *Lactobacillus plantarum (L. plantarum)* or *Lactobacillus casei* (*L. casei*) before treatment of pathogenic vesicles such as *E. coli* EVs to evaluate immunomodulatory and anti-inflammatory effects of *Lactobacillus* casei-derived vesicles (PC: positive control; LP: *Lactobacillus plantarum* EVs; LC: *Lactobacillus casei* EVs).

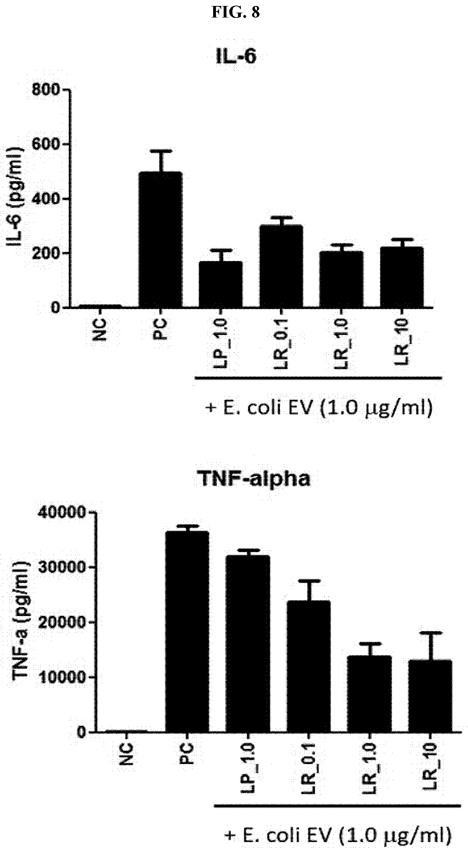

FIG. 8 is a result of evaluating an effect on the secretion of IL-6 and TNF-α which inflammatory mediators caused by *E. coli* EVs by pretreating vesicles derived from *Lactobacillus plantarum (L. plantarum)* or *Lactobacillus rhamnosus* (*L. rhamnosus*) before treatment of pathogenic vesicles such as *E. coli* EVs to evaluate immunomodulatory and anti-inflammatory effects of *Lactobacillus rhamnosus-derived* vesicles (PC: positive control; LP: *Lactobacillus plantarum* EVs; LR: *Lactobacillus rhamnosus* EVs).

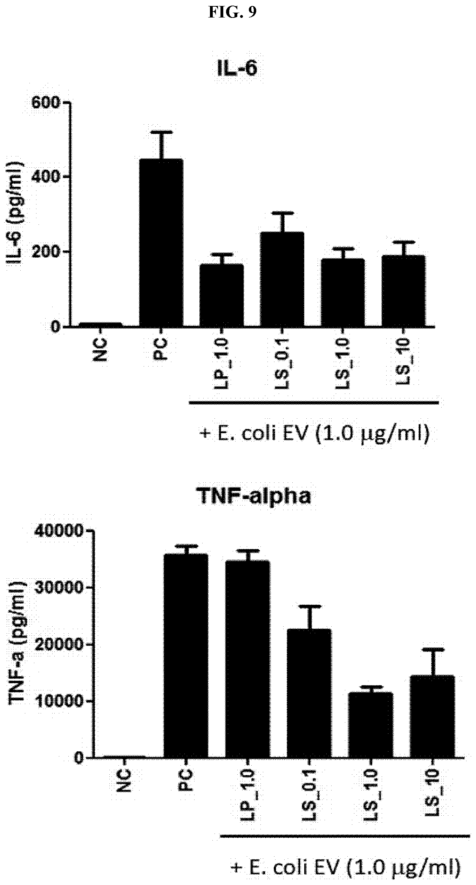

FIG. 9 is a result of evaluating an effect on the secretion of IL-6 and TNF-α which inflammatory mediators caused by *E. coli* EVs by pretreating vesicles derived from *Lactobacillus plantarum (L. plantarum)* or *Lactobacillus sakei (L. sakei*) before treatment of pathogenic vesicles such as *E. coli* EVs to evaluate immunomodulatory and anti-inflammatory effects of *Lactobacillus sakei-derived* vesicles (PC: positive control; LP: *Lactobacillus plantarum* EVs; LS: *Lactobacillus sakei* EVs).

### [Best Modes]

The present invention relates to vesicles derived from bacteria of the genus *Lactobacillus* and a use thereof.

The inventors confirmed that vesicles derived from bacteria of the genus *Lactobacillus* are significantly reduced in samples of patients with stomach cancer, renal failure, dementia and stroke, compared with a normal individual, through metagenomic analysis, thereby diagnosing a disease. In addition, as vesicles were isolated from *Lactobacillus brevis, Lactobacillus casei, Lactobacillus rhamnosus* and *Lactobacillus sakei* strains, which belong to bacteria of the genus *Lactobacillus* and their characteristics were identified, it was confirmed that the vesicles can be used for a composition for preventing or treating the disease.

Thus, the present invention provides a method of providing information for diagnosing stomach cancer, renal failure, dementia or stroke, the method comprising the following steps:
(a) extracting DNAs from extracellular vesicles isolated from samples of a normal individual and a subject;
(b) performing polymerase chain reaction (PCR) on the extracted DNA using a pair of primers prepared based on a gene sequence present in 16S rDNA to obtain each PCR product; and
(c) classifying a case in which a content of extracellular vesicles derived from bacteria of the genus *Lactobacillus* is lower than that of the normal individual sample, as stomach cancer, renal failure, dementia or stroke, through quantitative analysis of the PCR product.

The term "diagnosis" as used herein refers to determination of a condition of a disease of a patient over all aspects, in a broad sense. The contents of the determination are the disease entity, the etiology, the pathogenesis, the severity, the detailed aspects of a disease, the presence and absence of complications, the prognosis, and the like. The diagnosis in the present invention means determining whether stomach cancer, renal failure, dementia and stroke occur, the level of the disease, and the like.

The term "metagenome" as used herein also refers to a microbiome, and refers to a total of genomes including all viruses, bacteria, fungi, and the like in an isolated region such as soil and an animal's intestines, and is typically used as a concept of genomes explaining identification of a large number of microorganisms at one time by using a sequence analyzer in order to analyze uncultivated microorganisms. In particular, the metagenome does not refer to a genome of one species, but refers to a kind of mixed genome as a genome of all species of one environmental unit. The metagenome is, when one species is defined in the development process of omics biology, a term derived from the viewpoint of making a complete species is made by various species interacting with each other as well as one kind of functionally existing species. Technically, the metagenome is an object of a technique to identify all species in one environment and investigate interactions and metabolism by analyzing all DNAs and RNAs regardless of species using a rapid sequence analysis method.

In the present invention, the sample may be blood, but is not limited thereto.

Another aspect of the present invention provides a composition for preventing, treating or alleviating an inflammatory disease including stomach cancer, renal failure, dementia, stroke, an inflammatory skin disease, or the like, comprising vesicles derived from bacteria of the genus *Lactobacillus* as an active ingredient. The composition includes a food composition, a cosmetic composition and a pharmaceutical composition, and in the present invention, the food composition includes a health functional food composition. The composition of the present invention may be a formulation of an oral spray or inhalant.

The term "vesicles" used herein refers to structures formed with a nano-scale membrane, which are secreted from various bacteria. Vesicles derived from gram-positive bacteria such as *Lactobacillus* include peptidoglycan and lipoteichoic acid, which are components of the bacterial cell wall, and various low-molecular compounds in the vesicle, as well as proteins and nucleic acids. In the present invention, nanovesicles or vesicles are naturally secreted or artificially produced from bacteria of the genus *Lactobacillus,* and have an average diameter of 10 to 200 nm.

The vesicles may be isolated from a culturing solution comprising bacteria of the genus *Lactobacillus* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by a filter, gel filtration chromatography, free-flow electrophoresis, and capillary electrophoresis. Further, a process such as washing for removing impurities and concentration of obtained vesicles may be further included.

The term "prevention" as used herein refers to all actions that suppress an inflammatory disease including stomach cancer, renal failure, dementia, stroke, an inflammatory skin disease, and/or the like or delay the onset thereof via administration of the composition according to the present invention.

The term "treatment" as used herein refers to all actions that alleviate or beneficially change symptoms of an inflammatory disease including stomach cancer, renal failure, dementia, stroke, an inflammatory skin disease, and/or the like via administration of composition according to the present invention.

The term "alleviation" used as used herein refers to all actions that at least reduce a parameter associated with a condition to be treated, for example, the degree of symptoms.

The term "inflammatory disease" used herein may be a disease caused by a direct reaction of a humoral mediator constituting an immune system, or by a chain of bioreactions, which are generated by stimulating a local or systemic effector system, and the inflammatory disease in the present invention may be stomach cancer, renal failure, dementia, stroke or an inflammatory skin disease, and a disease accompanied by immune dysfunction, but the present invention is not limited thereto.

The pharmaceutical composition of the present invention may include a pharmaceutically acceptable carrier. The pharmaceutically acceptable carrier is typically used in formulation, and includes saline, sterile water, Ringer's solution, buffered saline, cyclodextrin, a dextrose solution, a maltodextrin solution, glycerol, ethanol, liposomes, and the like, but is not limited thereto, and may further include other typical additives such as an antioxidant and a buffer, if necessary. Further, the composition may be formulated into an injectable formulation, such as an aqueous solution, a suspension, and an emulsion, a pill, a capsule, a granule, or a tablet by additionally adding a diluent, a dispersant, a surfactant, a binder, a lubricant, and the like. With regard to suitable pharmaceutically acceptable carriers and formulations, the composition may be preferably formulated according to each ingredient by using the method disclosed in the Remington's literature. The pharmaceutical composition of the present invention is not particularly limited in formulation, but may be formulated into an injection, an inhalant, an external preparation for skin, an oral ingestion, or the like.

The pharmaceutical composition of the present invention may be administered orally or parenterally (e.g., intravenously, subcutaneously, intradermally, intranasally or intratracheally) according to a desired method, and a dose may vary according to the condition and body weight of a patient, the severity of a disease, a drug formulation, an administration route, and duration, but may be suitably selected by those of ordinary skill in the art.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields. The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

Specifically, an effective amount of the pharmaceutical composition according to the present invention may vary according to a patient's age, gender and body weight, and generally, the pharmaceutical composition may be administered at 0.001 to 150 mg, and preferably, 0.01 to 100 mg per kg of body weight daily or every two days, or 1 to 3 times daily. However, as the dose may be increased or decreased by an administration route, the severity of obesity, gender, a body weight or an age, the above-mentioned dose does not limit the scope of the present invention in any way.

The food composition of the present invention includes a health functional food composition. The food composition according to the present invention may be used by adding an active ingredient as is to food or may be used together with other foods or food ingredients, but may be appropriately used according to a typical method. The mixed amount of the active ingredient may be suitably determined depending on the purpose of use thereof (for prevention or alleviation). In general, when a food or beverage is prepared, the composition of the present invention is added in an amount of 15 wt% or less, preferably 10 wt% or less based on the raw materials. However, for long-term intake for the purpose of health and hygiene or for the purpose of health control, the amount may be less than the above-mentioned range.

Other ingredients are not particularly limited, except that the food composition of the present invention contains the active ingredient as an essential ingredient at the indicated ratio, and the food composition of the present invention may contain various flavorants, natural carbohydrates, and the like, like a typical beverage, as an additional ingredient. Examples of the above-described natural carbohydrate include common sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As the flavorant other than those described above, a natural flavorant (thaumatin, stevia extract, for example, rebaudioside A, glycyrrhizin and the like), and a synthetic flavorant (saccharin, aspartame and the like) may be advantageously used. The proportion of the natural carbohydrate may be appropriately determined by the choice of those of ordinary skill in the art.

The food composition of the present invention may contain various nutrients, vitamins, minerals (electrolytes), flavoring agents such as synthetic flavoring agents and natural flavoring agents, colorants and fillers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in a carbonated beverage, or the like, in addition to the additives. These ingredients may be used either alone or in combinations thereof. The ratio of these additives may also be appropriately selected by those of ordinary skill in the art.

The cosmetic composition of the present invention may include components conventionally used in a cosmetic composition, in addition to vesicles derived from bacteria of the genus *Lactobacillus,* and such components may include, for example, conventional additives including an antioxidant, a stabilizer, a solubilizer, a vitamin, a pigment, and a fragrance, and a carrier.

In addition, the composition of the present invention may be used in combination with an organic sunscreen which is conventionally used without degrading a skin protective effect by reaction with vesicles derived from bacteria of the genus *Lactobacillus,* in addition to the vesicles derived from bacteria of the genus *Lactobacillus.* As the organic sunscreen, one or more types selected from the group consisting of glyceryl PABA, drometrizole trisiloxane, drometrizole, digalloyl trioleate, disodium phenyl dibenzimidazole tetrasulfonate, diethylhexyl butamidotriazone, diethylamino hydroxybenzoyl hexylbenzoate, DEA-methoxycinnamate, a Lawson/dihydroxyacetone mixture, methylenebis-benzotriazolyltetramethylbutylphenol, 4-methylbenzylidene camphor, methyl anthranilate, benzophenone-3(oxybenzone), benzophenone-4, benzophenone-8(dioxyphebenzone), butyl methoxydibenzoylmethane, bisethylhexyloxyphenol methoxyphenyl triazine, cinoxate, ethyl dihydroxypropyl PABA, octocrylene, ethylhexyldimethyl PABA, ethylhexyl methoxycinnamate, ethylhexyl salicylate, ethylhexyl triazone, isoamyl-p-methoxycinnamate, polysilicon-15 (dimethicodiethylbenzal malonate), terephthalylidene dicamphor sulfonic acid and a salt thereof, TEA-salicylate and aminobenzoic acid (PABA), may be used.

Products that can contain the cosmetic composition of the present invention include, for example, cosmetics such as an astringent, a skin toner, a nourishing toner, various types of creams, essences, packs and foundations, cleansers, face washes, soaps, treatments, and tonics. Specific formulations of the cosmetic composition of the present invention include a skin lotion, a skin softener, a skin toner, an astringent, a lotion, a milk lotion, a moisturizing lotion, a nourishing lotion, a massage cream, a nourishing cream, a moisturizing cream, a hand cream, an essence, a nourishing essence, a pack, a soap, a shampoo, a cleansing foam, a cleansing lotion, a cleansing cream, a body lotion, a body cleanser, an emulsion, a lipstick, a makeup base, a foundation, a pressed powder, a loose powder, and an eyeshadow.

In one embodiment of the present invention, as a result of orally administering bacteria and bacteria-derived vesicles to mice and observing *in vivo* absorption, distribution, and excretion patterns of the bacteria and the vesicles, it was confirmed that, while the bacteria were not absorbed via the intestinal mucous membrane, the bacteria-derived vesicles were absorbed within 5 minutes after administration and systemically distributed, and excreted via the kidneys, liver, and the like (see Example 1).

In another embodiment of the present invention, a bacterial metagenomic analysis was performed by using the blood of patients with stomach cancer, renal failure, dementia and stroke and normal individuals. As a result, it was confirmed that vesicles derived from bacteria of the genus *Lactobacillus* were significantly decreased in blood of patients with stomach cancer, renal failure, dementia and stroke as compared to blood of normal individuals (see Examples 3 to 6).

In another embodiment of the present invention, the immunomodulatory and anti-inflammatory effects of *Lactobacillus brevis* strain-derived vesicles were evaluated, and as a result of evaluating the secretion of immune mediators after macrophages were treated with *Lactobacillus plantarum-derived* vesicles or various concentrations of the *Lactobacillus* brevis-derived vesicles before treatment with E. coli-derived vesicles, which are pathogenic vesicles, it was confirmed that the *Lactobacillus* brevis-derived vesicles effectively inhibit TNF-α secretion caused by the *E. coli*-derived vesicles, compared with *Lactobacillus plantarum-derived* vesicles (see Examples 7 and 8).

In another embodiment of the present invention, the immunomodulatory and anti-inflammatory effects of *Lactobacillus casei* strain-derived vesicles were evaluated, and as a result of evaluating the secretion of immune mediators after macrophages were treated with *Lactobacillus plantarum-derived* vesicles or various concentrations of the *Lactobacillus casei-*derived vesicles before treatment with *E. coli*-derived vesicles, which are pathogenic vesicles, it was confirmed that the *Lactobacillus* casei-derived vesicles effectively inhibit TNF-α secretion caused by the E. coli-derived vesicles, compared with *Lactobacillus plantarum-*derived vesicles (see Examples 7 and 9).

In another embodiment of the present invention, the immunomodulatory and anti-inflammatory effects of *Lactobacillus rhamnosus* strain-derived vesicles were evaluated, and as a result of evaluating the secretion of immune mediators after macrophages were treated with *Lactobacillus plantarum-derived* vesicles or various concentrations of the *Lactobacillus rhamnosus-derived* vesicles before treatment with E. coli-derived vesicles, which are pathogenic vesicles, it was confirmed that the *Lactobacillus rhamnosus-derived* vesicles effectively inhibit TNF-α secretion caused by the *E. coli*-derived vesicles, compared with *Lactobacillus plantarum*-derived vesicles (see Examples 7 and 10).

In another embodiment of the present invention, the immunomodulatory and anti-inflammatory effects of *Lactobacillus sakei* strain-derived vesicles were evaluated, and as a result of evaluating the secretion of immune mediators after macrophages were treated with *Lactobacillus plantarum-derived* vesicles or various concentrations of the *Lactobacillus sakei-*derived vesicles before treatment with *E. coli*-derived vesicles, which are pathogenic vesicles, it was confirmed that the *Lactobacillus sakei-derived* vesicles effectively inhibit TNF-α secretion caused by the *E. coli*-derived vesicles, compared with *Lactobacillus plantarum-*derived vesicles (see Examples 7 and 11).

Hereinafter, preferred Examples for helping the understanding of the present invention will be suggested. However, the following Examples are provided only to more easily understand the present invention, and the contents of the present invention are not limited by the following Examples.

### [Modes of the Invention]

### [Examples]

### Example 1. Analysis of in vivo Absorption, Distribution, and Excretion Patterns of Intestinal Bacteria and Vesicles Derived from Bacteria

In order to evaluate whether intestinal bacteria and bacteria-derived vesicles were systemically absorbed through the gastrointestinal tract, an experiment was performed with the following method. First, a dose of 50 µg of each of fluorescence-labeled intestinal bacteria and intestinal bacteria-derived vesicles was administered through the gastrointestinal tract to the stomach of a mouse, and fluorescence was measured after 0 minute, 5 minutes, 3 hours, 6 hours, and 12 hours. As a result of observing the entire image of the mouse, as illustrated in FIG. 1A, the bacteria were not systemically absorbed, but the vesicles derived from bacteria were systemically absorbed 5 minutes after administration, and fluorescence was strongly observed in the bladder 3 hours after administration, so that it could be seen that the vesicles were excreted to the urinary tract. Further, it could be seen that the vesicles were present in the body until 12 hours after administration.

In addition, in order to evaluate the pattern in which the intestinal bacteria and the vesicles derived from the intestinal bacteria infiltrated into various organs after they were systemically absorbed, 50 µg of bacteria and vesicles derived from bacteria labeled with fluorescence were administered in the same manner as described above, and then the urine, heart, lungs, liver, kidneys, spleen, fat, and muscle were collected 12 hours after administration. As a result of observing fluorescence in the collected tissues, as illustrated in FIG. 1B, it could be seen that the vesicles derived from bacteria were distributed in the urine, heart, lungs, liver, spleen, fat, muscle, and kidneys but the bacteria were not absorbed.

### Example 2. Metagenomic Analysis of Vesicles Derived from Bacteria in Clinical Sample

After blood was first put into a 10-ml tube and suspended matter was allowed to settle by a centrifuge (3,500 x g, 10 min, 4°C), only the supernatant was transferred to a new 10-ml tube. After bacteria and impurities were removed by using a 0.22-µm filter, they were transferred to a Centriprep tube (centrifugal filters 50 kD) and centrifuged at 1,500 x g and 4°C for 15 minutes, materials smaller than 50 kD were discarded, and the residue was concentrated to 10 ml. After bacteria and impurities were removed once again by using a 0.22-µm filter, the supernatant was discarded by using a ultra-high speed centrifugation at 150,000 x g and 4°C for 3 hours with a Type 90Ti rotor, and an aggregated pellet was dissolved in physiological saline (PBS).

Internal DNA was extracted out of the lipid by boiling 100 µl of the vesicles isolated by the above method at 100°C, and then cooled on ice for 5 minutes. And then, in order to remove the remaining suspended matter, the DNA was centrifuged at 10,000 x g and 4°C for 30 minutes, and only the supernatant was collected. And, the amount of DNA was quantified by using Nanodrop. Thereafter, in order to confirm whether the DNA derived from bacteria was present in the extracted DNA, PCR was performed with 16s rDNA primers shown in the following Table 1 and it was confirmed that genes derived from bacteria were present in the extracted genes.

**[Table 1]**

| primer | | Sequence | SEQ ID No. |
|---|---|---|---|
| 16S rDNA | 16S_V3_F | 5'- TCGTCGGCAGCGTCAGATGTGTATAAGA GACAGCCTACGGGNGGCWGCAG-3' | 1 |
| | 16S_V4_R | 5'- GTCTCGTGGGCTCGGAGATGTGTATAAG | 2 |
| | | AGACAGGACTACHVGGGTATCTAATCC | |

The DNA extracted by the above method was amplified using the 16S rDNA primers, and then sequencing was performed (Illumina MiSeq sequencer), the results were output as a standard flowgram format (SFF) file, the SFF file was converted into a sequence file (.fasta) and a nucleotide quality score file using GS FLX software (v2.9), and then the reliability estimation for the reads was confirmed, and a portion in which the window (20 bps) average base call accuracy was less than 99% (Phred score<20) was removed. For the OTU (operational taxonomy unit) analysis, clustering was performed according to sequence similarity by using UCLUST and USEARCH, the genus, family, order, class, and phylum were clustered based on 94%, 90%, 85%, 80%, and 75% sequence similarity, respectively, classification was performed at the phylum, class, order, family, and genus levels of each OUT, and bacteria having a sequence similarity of 97% or more at the genus level were profiled by using the 16S RNA sequence database (108,453 sequences) of BLASTN and GreenGenes (QIIME).

### Example 3. Metagenomic analysis of bacteria-derived vesicles in blood of patient with stomach cancer and normal individual

After a metagenomic analysis was performed using the method of Example 2 on the blood from 66 patients with stomach cancer, and 198 normal individuals who were matched in age and sex by extracting genes from vesicles present in the blood, the distribution of vesicles derived from bacteria of the genus *Lactobacillus* was evaluated. As a result, it was confirmed that vesicles derived from bacteria of the genus *Lactobacillus* were significantly decreased in the blood from the patients with stomach cancer as compared to the blood from the normal individuals (see Table 2 and FIG. 2).

**[Table 2]**

| Blood | Control | | Stomach cancer | | t-test | |
|---|---|---|---|---|---|---|
| Taxon | Mean | SD | Mean | SD | p-value | Ratio |
| g_ Lactobacillus | 0.0325 | 0.0246 | 0.0242 | 0.0150 | 0.0015 | 0.75 |

### Example 4. Metagenomic analysis of bacteria-derived vesicles in blood of patient with renal failure and normal individual

After a metagenomic analysis was performed using the method of Example 2 on the blood from 21 patients with renal failure, and 19 normal individuals who were matched in age and sex by extracting genes from vesicles present in the blood, the distribution of vesicles derived from bacteria of the genus *Lactobacillus* was evaluated. As a result, it was confirmed that vesicles derived from bacteria of the genus *Lactobacillus* were significantly decreased in the blood from the patients with renal failure as compared to the blood from the normal individuals (see Table 3 and FIG. 3).

**[Table 3]**

| Blood | Control | | Renal failure | | t-test | |
|---|---|---|---|---|---|---|
| Taxon | Mean | SD | Mean | SD | p-value | Ratio |
| g_ Lactobacillus | 0.0552 | 0.0268 | 0.0034 | 0.0133 | <0.0001 | 0.06 |

### Example 5. Metagenomic analysis of bacteria-derived vesicles in blood of patient with dementia and normal individual

After a metagenomic analysis was performed using the method of Example 2 on the blood from 45 patients with dementia, and 49 normal individuals who were matched in age and sex by extracting genes from vesicles present in the blood, the distribution of vesicles derived from bacteria of the genus *Lactobacillus* was evaluated. As a result, it was confirmed that vesicles derived from bacteria of the genus *Lactobacillus* were significantly decreased in the blood from the patients with dementia as compared to the blood from the normal individuals (see Table 4 and FIG. 4).

**[Table 4]**

| Blood | Control | | Dementia | | t-test | |
|---|---|---|---|---|---|---|
| Taxon | Mean | SD | Mean | SD | p-value | Ratio |
| g_ Lactobacillus | 0.0557 | 0.0117 | 0.0313 | 0.0170 | 0.0051 | 0.56 |

### Example 6. Metagenomic analysis of bacteria-derived vesicles in blood of patient with stroke and normal individual

After a metagenomic analysis was performed using the method of Example 2 on the blood from 115 patients with stroke, and 109 normal individuals who were matched in age and sex by extracting genes from vesicles present in the blood, the distribution of vesicles derived from bacteria of the genus *Lactobacillus* was evaluated. As a result, it was confirmed that vesicles derived from bacteria of the genus *Lactobacillus* were significantly decreased in the blood from the patients with stroke as compared to the blood from the normal individuals (see Table 5 and FIG. 5).

**[Table 5]**

| Blood | Control | | Stroke | | t-test | |
|---|---|---|---|---|---|---|
| Taxon | Mean | SD | Mean | SD | p-value | Ratio |
| g_ Lactobacillus | 0.0361 | 0.0354 | 0.0046 | 0.0225 | <0.0001 | 0.13 |

### Example 7. Isolation of vesicles from culture of bacteria of the genus Lactobacillus

*Lactobacillus brevis, Lactobacillus casei, Lactobacillus rhamnosus,* and *Lactobacillus sakei* strains were isolated from environmental samples and cultured, and then vesicles were isolated therefrom, followed by analysis of their characteristics. Each *Lactobacillus* strain was incubated in a de Man, Rogosa and Sharpe (MRS) medium until an absorbance (OD₆₀₀) reached 1.0 to 1.5 at 37 °C under an aerobic condition, and then sub-cultured in a Luria-Bertani (LB) medium. Subsequently, the medium containing the strain was recovered, and then centrifuged at 10,000 g and 4 °C for 20 minutes to remove the strain, followed by filtration through a 0.22 µm filter. The filtered supernatant was concentrated to a volume of 50 mL through a 100 kDa Pellicon 2 Cassette filter membrane (Merck Millipore, US) through microfiltration using a MasterFlex pump system (Cole-Parmer, US). The concentrated supernatant was filtered again using a 0.22 µm filter. Afterward, a protein was quantified by BCA assay, and the following experiment was performed on the obtained vesicles.

### Example 8. Immunomodulatory and anti-inflammatory effects of Lactobacillus brevis-derived vesicles

To evaluate the immunomodulatory and anti-inflammatory effects of *Lactobacillus* brevis-derived vesicles, a macrophage cell line was pre-treated with various concentrations (0.1, 1 and 10 µg/mL) of the *Lactobacillus* brevis-derived vesicles for 12 hours, and then treated with 1 µg/mL of E. coli-derived vesicles, which are pathogenic vesicles. After 12 hours, the secretion of inflammatory cytokines was measured by ELISA. To perform ELISA, capture antibodies were diluted in phosphate buffered saline (PBS), and then 50 µL of the resulting dilution was dispensed to a 96-well polystyrene plate according to a working concentration, followed by an overnight reaction at 4 °C.

Subsequently, the resultant was washed three times with 100 µL of a 0.05% Tween-20-containing PBS (PBST) solution, 100 µL of a 1% BSA-containing PBS (RD) solution was dispensed for blocking at room temperature for 1 hour, and then 50 µL each of the sample and the standard was dispensed according to a concentration, followed by a reaction at room temperature for 2 hours. Following washing with 100 µL of PBST three times, detection antibodies were diluted in RD and then dispensed at 50 µL according to a working concentration to perform a reaction at room temperature for 2 hours. The resultant was washed three times with 100 µL of PBST, and Streptavidin-HRP (R&D Systems, USA) was diluted in RD at 1/40 and dispensed at 50 µL, followed by a reaction at room temperature for 20 minutes. Finally, the resultant was washed three times with 100 µL of PBST, 50 µL of a TMB substrate (SurModics, USA) was dispensed, and when color development progressed for 5 to 20 minutes, 50 µL of a 1M sulfuric acid solution was dispensed to stop the reaction, and then absorbance was measured at 450 nm using a SpectraMax M3 microplate reader (Molecular Devices, USA).

As a result, as shown in FIG. 6, by the pre-treatment of *Lactobacillus brevis-derived* vesicles, it was confirmed that the secretion of IL-6 and TNF-α caused by E. coli-derived vesicles was significantly inhibited. Particularly, by the pre-treatment of the *Lactobacillus* brevis-derived vesicles, it was confirmed that the effect of inhibiting TNF-α secretion significantly increases, compared with *Lactobacillus plantarum,* which is an effective microorganism control.

### Example 9. Immunomodulatory and anti-inflammatory effects of Lactobacillus casei-derived vesicles

To evaluate the immunomodulatory and anti-inflammatory effects of *Lactobacillus* casei-derived vesicles, a macrophage cell line was pre-treated with various concentrations (0.1, 1 and 10 µg/mL) of the *Lactobacillus* casei-derived vesicles for 12 hours, and then treated with 1 µg/mL of E. coli-derived vesicles, which are pathogenic vesicles. After 12 hours, the secretion of inflammatory cytokines was measured by ELISA. As a result, by the pre-treatment of *Lactobacillus* casei-derived vesicles, it was confirmed that the secretion of IL-6 and TNF-α caused by E. coli-derived vesicles was significantly inhibited (see FIG. 7). Particularly, by the pre-treatment of the *Lactobacillus* casei-derived vesicles, it was confirmed that the effect of inhibiting TNF-α secretion significantly increases, compared with *Lactobacillus plantarum,* which is an effective microorganism control (see FIG. 7).

### Example 10. Immunomodulatory and anti-inflammatory effects of Lactobacillus rhamnosus-derived vesicles

To evaluate the immunomodulatory and anti-inflammatory effects of *Lactobacillus rhamnosus-derived* vesicles, a macrophage cell line was pre-treated with various concentrations (0.1, 1 and 10 µg/mL) of the *Lactobacillus rhamnosus-derived* vesicles for 12 hours, and then treated with 1 µg/mL of E. coli-derived vesicles, which are pathogenic vesicles. After 12 hours, the secretion of inflammatory cytokines was measured by ELISA. As a result, as shown in FIG. 8, by the pre-treatment of *Lactobacillus rhamnosus-derived* vesicles, it was confirmed that the secretion of IL-6 and TNF-α caused by E. coli-derived vesicles was significantly inhibited. Particularly, by the pre-treatment of the *Lactobacillus rhamnosus-*derived vesicles, it was confirmed that the effect of inhibiting TNF-α secretion significantly increases, compared with *Lactobacillus plantarum,* which is an effective microorganism control.

### Example 11. Immunomodulatory and anti-inflammatory effects of Lactobacillus sakei-derived vesicles

To evaluate the immunomodulatory and anti-inflammatory effects of *Lactobacillus sakei-derived* vesicles, a macrophage cell line was pre-treated with various concentrations (0.1, 1 and 10 µg/mL) of the *Lactobacillus sakei-derived* vesicles for 12 hours, and then treated with 1 µg/mL of E. coli-derived vesicles, which are pathogenic vesicles. After 12 hours, the secretion of inflammatory cytokines was measured by ELISA. As a result, as shown in FIG. 9, by the pre-treatment of *Lactobacillus sakei*-derived vesicles, it was confirmed that the secretion of IL-6 and TNF-α caused by *E. coli*-derived vesicles was significantly inhibited. Particularly, by the pre-treatment of the *Lactobacillus sakei*-derived vesicles, it was confirmed that the effect of inhibiting TNF-α secretion significantly increases, compared with *Lactobacillus plantarum,* which is an effective microorganism control.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [industrial Applicability]

Since vesicles derived from bacteria of the genus *Lactobacillus* according to the present invention may be used in a method of diagnosing stomach cancer, renal failure, dementia or stroke, and a composition for preventing, alleviating or treating the disease, they are expected to be effectively used in related medical, cosmetic and food industry fields.

## Claims

1. A method of providing information for diagnosing stomach cancer, renal failure, dementia or stroke, the method comprising the following steps:
(a) extracting DNAs from extracellular vesicles isolated from samples of a normal individual and a subject;
(b) performing polymerase chain reaction (PCR) on the extracted DNA using a pair of primers prepared based on a gene sequence present in 16S rDNA to obtain each PCR product; and
(c) classifying a case in which a content of extracellular vesicles derived from bacteria of the genus *Lactobacillus* is lower than that of the normal individual sample, as stomach cancer, renal failure, dementia or stroke, through quantitative analysis of the PCR product.

2. The method of claim 1, wherein the sample in Step (a) is blood.

3. A pharmaceutical composition for preventing or treating an inflammatory disease accompanied by immune dysfunction, comprising vesicles derived from bacteria of the genus *Lactobacillus* as an active ingredient.

4. The pharmaceutical composition of claim 3, wherein the vesicles have an average diameter of 10 to 200 nm.

5. The pharmaceutical composition of claim 3, wherein the vesicles are secreted naturally or artificially from bacteria of the genus *Lactobacillus.*

6. The pharmaceutical composition of claim 3, wherein the vesicles derived from bacteria of the genus *Lactobacillus* are one or more vesicles selected from the group consisting of *Lactobacillus brevis*-derived vesicles, *Lactobacillus casei*-derived vesicles, *Lactobacillus rhamnosus-derived* vesicles and *Lactobacillus sakei*-derived vesicles.

7. The pharmaceutical composition of claim 3, wherein the inflammatory disease accompanied by immune dysfunction is one or more diseases selected from the group consisting of stomach cancer, renal failure, dementia and stroke.

8. A food composition for preventing or alleviating an inflammatory disease accompanied by immune dysfunction, comprising vesicles derived from bacteria of the genus *Lactobacillus* as an active ingredient.

9. The food composition of claim 8, wherein the vesicles derived from bacteria of the genus *Lactobacillus* are one or more vesicles selected from the group consisting of *Lactobacillus* brevis-derived vesicles, *Lactobacillus* casei-derived vesicles, *Lactobacillus rhamnosus-derived* vesicles and *Lactobacillus* sakei-derived vesicles.

10. The food composition of claim 8, wherein the inflammatory disease accompanied by immune dysfunction is one or more diseases selected from the group consisting of stomach cancer, renal failure, dementia and stroke.

11. A cosmetic composition for preventing or alleviating an inflammatory skin disease, comprising vesicles derived from bacteria of the genus *Lactobacillus* as an active ingredient.

12. The cosmetic composition of claim 11, wherein the vesicles derived from bacteria of the genus *Lactobacillus* are one or more vesicles selected from the group consisting of *Lactobacillus* brevis-derived vesicles, *Lactobacillus* casei-derived vesicles, *Lactobacillus rhamnosus-derived* vesicles and *Lactobacillus* sakei-derived vesicles.

13. A method of diagnosing stomach cancer, renal failure, dementia or stroke, the method comprising the following steps:
(a) extracting DNAs from extracellular vesicles isolated from samples of a normal individual and a subject;
(b) performing polymerase chain reaction (PCR) on the extracted DNA using a pair of primers prepared based on a gene sequence present in 16S rDNA to obtain each PCR product; and
(c) determining a case in which a content of extracellular vesicles derived from bacteria of the genus *Lactobacillus* is lower than that of the normal individual sample, as stomach cancer, renal failure, dementia or stroke, through quantitative analysis of the PCR product.

14. A method of preventing or treating an inflammatory disease accompanied by immune dysfunction, the method comprising a step of administering a pharmaceutical composition comprising vesicles derived from bacteria of the genus *Lactobacillus* as an active ingredient to a subject.

15. The method of claim 14, the inflammatory disease is one or more diseases selected from the group consisting of stomach cancer, renal failure, dementia and stroke.

16. A use of vesicles derived from bacteria of the genus *Lactobacillus* for preventing or treating an inflammatory disease accompanied by immune dysfunction.

17. The use of claim 16, the inflammatory disease is one or more diseases selected from the group consisting of stomach cancer, renal failure, dementia and stroke.
